# EUROPEAN PATENT SPECIFICATION

(11) **EP 1 187 917 B1**
(45) Date of publication and mention of the grant of the patent: **07.10.2009**
(21) Application number: 00935449.9
(22) Date of filing: 07.06.2000
(51) Int. Cl.: C12N 15/12, C07K 14/705, C07K 16/28, C12N 5/12, A61K 31/70, A61K 38/17, A61K 39/395, A61K 48/00, C12Q 1/68, G01N 33/68

(54) **CD44 SPLICE VARIANT ASSOCIATED WITH RHEUMATOID ARTHRITIS**
MIT RHEUMATISCHER ARTHRITIS ASSOZIIERTE SPLEISSVARIANTE VON CD44
VARIANTE D'EPISSAGE DE CD44 ASSOCIEE A LA POLYARTHRITE RHUMATOIDE

(30) Priority: 08.06.1999 IL 13035699; 21.12.1999 IL 13364799
(43) Date of publication of application: 20.03.2002
(73) Proprietor: Yissum Research Development Company of the Hebrew University of Jerusalem Ltd, 91042 Jerusalem (IL)
(72) Inventor: NAOR, David, 93503 Jerusalem (IL); GOLAN, Itshak, 77632 Ashdod (IL); NEDVETZKI, Shlomo, 93305 Jerusalem (IL)
(74) Representative: Vossius & Partner
(86) International application number: PCT/IL2000/000326
(87) International publication number: WO 2000/075312

(56) References cited:
- WO-A-94/09811
- WO-A-20/05007700
- DE-A- 19 708 713
- KUGELMAN L. C. ET AL.: "The core protein of epican, a heparan sulfate proteoglycan on keratinocytes, is an alternative form of CD44." JOURNAL OF INVESTIGATIVE DERMATOLOGY, vol. 99, no. 4, 1992, pages 381-385, XP000952612 ISSN: 0022-202X
- NAOR D. ET AL.: "CD44: Structure, function, and association with the malignant process." ADVANCES IN CANCER RESEARCH, vol. 71, 1997, pages 241-319, XP000952636 ISBN: 0-12-006671-8 cited in the application
- DATABASE EMBL [Online] 1 July 1989 (1989-07-01), "tRNA ligase (EC 6.5.1.3)" retrieved from EBI Database accession no. P09880
- DATABASE EMBL [Online] 1989, "Uncharacterized GTP-binding protein C1B3.04c." retrieved from EBI Database accession no. O13869

## Description

### FIELD OF THE INVENTION

The present invention concerns a novel nucleic acid sequence encoding a CD44 variant and which comprises the three additional nucleotides CAG resulting in an insertion of a codon for the amino acid alanine at the 5'-end of exon v5 of the CD44 gene, vectors and host cells comprising said sequence, amino acid sequences encoded by said sequence, antibodies reactive with said amino acid sequences and pharmaceutical compositions comprising any of the above. The invention also concerns methods for the diagnosis of rheumatoid arthritis (RA).

### PRIOR ART

The following is a list of references which are intended for better understanding of the background of the present invention:

### LIST OF PRIOR ART

Aune, T.M., et al., Published EP Application No. 501233 (1992).
Hale, L.P, et al., WO 9409811 (1994)/
Herrlich *et al.,* European Patent No. 538754, (1991).
Jalkanen, S., et al., WO 9500658, (1993).
Naor, D., et al., Adv. Cancer Res., 71:241, (1997).
Screaton, G.R., et al., Proc. Natl. Acad. Sci. USA, 89:12160, (1992).
Verdrengh, M., et al., Scand J. Immunol., 42:353, (1995)

### BACKGROUND OF THE INVENTION

The cell surface adhesion molecule, designated CD44, has been shown to be implicated in cell-cell and cell-matrix interactions, as well as in cell traffic, cell transendothelial migration, and cell homing through the high endothelial venule (HEV). CD44 is a single chain molecule comprising a conserved aminoterminal extracellular domain, a nonconserved membrane proximal region, a variable region expressing various combinations of variant exons, a conserved transmembrane spanning domain and a conserved cytoplasmic tail. The genomic map of CD44 includes 5 constant exons at the 5' terminus, 5 constant exons in the 3' end. The mouse CD44 gene includes also 10 variant exons in the middle of the molecule designated V₁-V₁₀ resulting in a total of 20 exons. The human CD44 gene comprises only 9 of these 10 variant exons (V₂-V₁₀) thus comprising a total of 19 exons (Screaton, G.R., *et al.,* 1992). Differential alternative splicing generates many isoforms of CD44 that express various combinations of variant exons (designated Exon Vx, x = 1-10), which are inserted in the membrane proximal domain and constitute the variable region of the molecule. These molecules are designated CD44 variants (CD44v). To date, 20 isoforms of CD44 are known.

Standard CD44 (CD44s), which lacks the entire variable region, and is expressed predominantly on hematopoietic cells is, therefore, also known as hematopoietic CD44 (CD44H). The CD44 N-terminus contains the ligand binding site of the molecule. Hyaluronic acid (HA) is the principal ligand of CD44, but other ECM components (laminin, collagen, fibronectin and chondroitin sulfate) as well as non-ECM constituents (mucosal vascular addressin, serglycin, osteopontin and class II invariant chain) can also interact with the CD44 receptor. Marked accumulation of CD44, and sometimes hyaluronic acid, is detected in areas of intensive cell migration and cell proliferation, as in wound healing, tissue remodeling, inflammation (including autoinflammation), morphogenesis and carcinogenesis

The involvement of CD44 protein and variants thereof in autoimmune diseases is known. For example, it has been shown that anti-CD44 monoclonal antibodies (mAbs) can ameliorate the severity of experimentally induced autoimmune arthritis in mice (Verdrengh. M. *et al.,* 1995). However, these mAbs are directed against the constant region of CD44 (and are thus designated anti-pan CD44 mAbs). Such mAbs may also block normal CD44 which is required for migratory activity of normal immune and inflammatory cells.

Monoclonal Abs directed against various variant regions of CD44 have also been suggested as potential agents for treatment of autoimmune diseases. Herrlich *et al.,* describe mAbs directed against metastasis-specific variants of CD44v surface protein of a rat pancreatic adenocarcinoma (Herrlich *et al.,* 1991). These antibodies are proposed for producing immunosuppression for therapeutic treatment of immuno regulatory disorders including, for example, diseases of the rheumatic type. Monoclonal antibodies reactive with CD44 which inhibit T-cell proliferation were also provided for treatment of various autoimmune diseases (Aune, T.M., *et al.,* 1992). Monoclonal antibodies binding to forms of CD44 containing the exon v6 peptide were also reported as being useful for diagnosing inflammatory diseases (Jalkanen, S., *et al.,* 1994). In addition, it has been reported (Hale, L.P., *et al.,* 1992) that administration of a CD44 protein or peptide or derivative can be used for treating various autoimmune diseases.

The involvement of CD44 in malignant processes has also been described (Naor, D., 1997). Anti-CD44 mAbs which were injected into mice, were shown to inhibit or prevent infiltration of various lymphoma and carcinoma cells into their target organs. In addition, transfection of a variant CD44 isoform into non metastatic rat pancreatic adenocarcinoma cells conferred metastatic potential to these cells.

### GLOSSARY

The following are terms which will be used throughout the description and claims and which should be understood in accordance with the invention to mean as follows: ***"Rheumatoid Arthritis-CD44 (RA-CD44) variant nucleic acid cording sequence "*** interchangeably referred to also as the **"*RA-CD44 variant coding sequence*"** or **"*RA-CD44 variant*"** *-* refers to nucleic acid molecules having the sequence shown in SEQ ID NO: 1, nucleic acid molecules having at least *90% identity* (see below) to said sequence and *fragments* (see below) of the above molecules of least 20 nucleotides long. These molecules comprise sequences coding for a novel, naturally occurring, alternative splice variant of the native and known CD44 transcript. It should be emphasized that a novel variant of the present invention is a naturally occurring mature mRNA sequence resulting from alternative splicing of the primary mRNA transcript and not merely a truncated, mutated or fragmented form of the known sequence.

This RA-CD44 variant sequence comprises Exons 1-5, 15-17 and 19 of the constant part of the CD44 gene as well as Exons 7-14 (v3-v10) of the variable region of the gene (Screaton et al, *supra*). The variant coding sequence comprises three additional bases (CAG) at the 5' end of Exon v5 as explained below.

***"RA-CD44 Variant product - also referred at times as "variant product, " "RA-CD44 variant protein," "variant protein" "RA-CD44 variant peptide"*** or **" *variant peptide*"** *-* is a polypeptide having an amino acid sequence encoded by the RA-CD44 variant coding sequence. By "polypeptide" is intended a peptide or protein, as well as peptides or proteins having *chemically modified* amino acids (see below) such as a glycopeptide or glycoprotein. The amino acid sequence of a preferred RA-CD44 variant product is shown in SEQ ID NO: 2. "RA-CD44 variant product" also includes *homologues* (see below) of said amino acid sequence in which one or more amino acids has been added, deleted, *substituted* (see below) or *chemically modified* (see below) as well as *fragments* (see below) of this sequence having at least 6 amino acids.

**"*Nucleic acid molecule*" *or*** "***nucleic acid***" *-* a single-stranded or double-stranded polymer composed of DNA nucleotides, RNA nucleotides or a combination of both types and may include natural nucleotides, chemically modified nucleotides and synthetic nucleotides.

**"*Amino acid sequence*"** *-* a sequence composed of any one of the 20 naturally appearing amino acids, amino acids which have been *chemically modified* (see below), or synthetic amino acids.

***"Fragment of RA CD44 variant nucleic acid coding sequence"** -* a fragment of at least 20 nucleotides of a RA-CD44 variant nucleic acid coding sequence having the sequence of SEQ ID NO: 1, or a fragment of at least 20 nucleotides of a RA-CD44 nucleic acid coding sequence having a sequence that is 90% identical to the sequence of SEQ ID NO:1, which at least 20 nucleotides does not appear as a continuous stretch in the *original nucleic acid sequence* (see below). The fragment will preferably comprise at least 30 nucleotides, more preferably at least 50 nucleotides, most preferably at least 100 nucleotides of the RA-CD44 sequence. At a minimum, the fragment will comprise the region corresponding to the variant splice junction site which corresponds to nucleotides 908-910 in SEQ ID NO:1 (see explanation on pages 10 and 11 below). The fragment may be a sequence which was previously described in the context of the published CD44 RNA and which affects the amino acid sequence encoded by the known gene. In the case of the sequence of SEQ ID NO: 1, the variant nucleic acid sequence includes a sequence which was not included in the original CD44 sequence (a sequence which was an intron in the original sequence) and the fragment may be that additional sequence itself.

**"*****Fragment of RA-CD44 variant products"*** -fragment of at least 6 amino acids of the RA-CD44 variant polypeptide having the amino acid sequence of SEQ ID NO:2, or fragments of at least 6 amino acids of a homologue of said polypeptide. The fragments will preferably comprise at least 10 amino acids, more preferably at least 20 amino acids, most preferably at least 30 amino acids, of said RA-CD44 variant polypeptide, or said homologue. At a minimum, the fragment will comprise the region encoded by the variant splice junction site which corresponds to amino acid residue 303 in SEQ ID NO:2.

**"*Homologue of variant*"** *-* polypeptide having an amino acid sequence that is at least 90% identical to the sequence of SEQ ID NO: 2, or at least 90% identical to a fragment of at least 6 amino acids of the sequence of SEQ ID NO:2. The variation in amino acid sequence between the homologue and the sequence of SEQ ID NO: 2 or a fragment thereof, arises from the addition, deletion, substitution or chemical modification of one or more amino acids of the sequence of SEQ ID NO:2. Where the homologue contains a substitution, the substitution is preferably a conservative one. The addition, deletion or substitution may be in regions or adjacent to regions where the RA-CD44 variant product differs from the *original protein sequence* (see below), however, a homologue will preferably retain the additional alanine residue (corresponding to residue 303 of SEQ ID NO:2) characteristic of the RA-CD44 variant product.

**"*Conservative substitution*"** *-* refers to the substitution of an amino acid in one class by an amino acid of the same class, where a class is defined by common physicochemical amino acid side chain properties and high substitution frequencies in homologous proteins found in nature, as determined, for example, by a standard Dayhoff frequency exchange matrix or BLOSUM matrix. [Six general classes of amino acid side chains have been categorized and include: Class I (Cys); Class II (Ser, Thr, Pro, Ala, Gly); Class III (Asn, Asp, Gln, Glu); Class IV (His, Arg, Lys); Class V (Ile. Leu, Val, Met); and Class VI (Phe, Tyr, Trp). For example, substitution of an Asp for another class III residue such as Asn, Gln, or Glu, is a conservative substitution.

**"*Non-conservative substitution*"** *-* refers to the substitution of an amino acid in one class with an amino acid from another class; for example, substitution of an Ala, a class II residue, with a class III residue such as Asp, Asn, Glu, or Gln,

**"*Chemically modified*"** *-* when referring to the product of the invention, means a product (protein) where at least one of its amino acid residues is modified either by natural processes, such as processing or other post-translational modifications, or by chemical modification techniques which are well known in the art. Among the numerous known modifications typical, but not exclusive examples include: acetylation, acylation, amidation, ADP-ribosylation, glycosylation, glycosaminoglycanation, GPI anchor formation, covalent attachment of a lipid or lipid derivative, methylation, myristlyation, pegylation, prenylation, phosphorylation, ubiqutination, or any similar process. A preferred modification for the polypeptides of the present invention is pegylation.

**"*Optimal alignment*"** *-* is defined as an alignment giving the highest percent identity score. Such alignment can be performed using a variety of commercially available sequence analysis programs, such as the local alignment program LALIGN using a ktup of 1, default parameters and the default PAM.

***"Having at least 90% identity"** -* with respect to two sets of amino acid or nucleic acid sequences, refers to the percentage of residues that are identical in the two sequences when the sequences are optimally aligned. Thus, at least 90% amino acid sequence identity means that at least 90% of the amino acids in two or more optimally aligned polypeptide sequences are identical, however this definition explicitly excludes sequences which are 100% identical with the original nucleic acid sequence or original protein sequence from which the variant of the invention was varied.

**"*Isolated nucleic acid molecule having a variant nucleic acid sequence*"** is a nucleic acid molecule that comprises the variant coding sequence. Said isolated nucleic acid molecule may include, but is not limited to, the variant coding sequence as an independent insert; may include the variant coding sequence fused to an additional coding sequence, encoding together a fusion protein in which the variant coding sequence is the dominant coding sequence (for example, the additional coding sequence may code for a signal peptide); the variant coding sequence may be in combination with non-coding sequences, e.g., introns or control elements, such as promoter and terminator elements or 5' and/or 3' untranslated regions, effective for expression of the coding sequence in a suitable host; or may be a vector in which the variant protein coding sequence is heterologous.

**"*Expression vector*"** *-* refers to vectors that have the ability to incorporate and express heterologous DNA fragments in a foreign cell. Many prokaryotic and eukaryotic expression vectors are known and/or commercially available. Selection of appropriate expression vectors is within the knowledge of those having skill in the art.

**"*Deletion*"** *-* is a change in either nucleotide or amino acid sequence in which one or more nucleotides or amino acid residues, respectively, are absent as compared to the naturally occurring sequence.

**"*Insertion*" *or* "*addition*"** *-* is that change in a nucleotide or amino acid sequence which has resulted in the addition of one or more nucleotides or amino acid residues, respectively, as compared to the naturally occurring sequence.

**"*Substitution*"** *-* replacement of one or more nucleotides or amino acids by different nucleotides or amino acids, respectively as compared to the naturally occurring sequence. As regards amino acid sequences, the substitution may be conservative or non-conservative.

**"*Antibody*"** *-* refers to antibodies of any of the classes IgG, IgM, IgD, IgA. and IgE antibody. The definition includes polyclonal antibodies or monoclonal antibodies. This term refers to whole antibodies or fragments of the antibodies comprising the antigen-binding domain of the anti-variant product antibodies, e.g. scFv, Fab, F(ab')2, other antibodies without the Fc portion, single chain antibodies, bispecific antibodies, diabodies, other fragments consisting of essentially only the variable, antigen-binding domain of the antibody, etc., which substantially retain the antigen-binding characteristics of the whole antibody from which they were derived.

**"*Agonist*"** *-* as used herein, refers to a molecule which mimics the effect of the natural RA-CD44 variant product or has enhanced activity compared with the natural RA-CD44 variant product, or at times even increases or prolongs the duration of the biological activity of said variant product, as compared to that induced by the variant product itself. The mechanism may be by any mechanism known to prolonging activities of biological molecules such as binding to receptors; prolonging the lifetime of the molecules; increasing the activity of the molecules on its target; increasing the affinity of molecules to its receptor; inhibiting degradation or proteolysis of the molecules, etc. Agonists may be polypeptides, nucleic acids, carbohydrates, lipids, or derivatives thereof, or any other molecules which can positively modulate the activity of the variant product.

**"*Antagonist*"** *-* refers to a molecule which inhibits or shortens the duration of the biological activity of the natural RA-CD44 variant product. This may be done by any mechanism known to deactivate or inhibit biological molecules such as blocking of the receptor, blocking of an active site, competition on a binding site, enhancement of degradation, etc. Antagonists may be polypeptides, nucleic acids, carbohydrates, lipids, or derivatives thereof, or any other molecules which can negatively modulate the activity of said product.

**"*Treating a disease*" -** refers to administering a therapeutic substance effective to ameliorate symptoms associated with a disease, to lessen the severity or cure the disease, or to prevent the disease from occurring.

**"*Detection*" *-*** refers, in some aspects of the invention, to a method of detection of a disease, disorder, pathological or normal condition. This term may refer to detection of a predisposition to a disease as well as for establishing the prognosis of the patient by determining the severity of the disease.

**"*Probe*" *-*** a nucleic acid molecule comprising the variant coding sequence, or a sequence complementary therewith, when used to detect presence of other similar sequences in a sample. The detection is carried out by identification of hybridization complexes between the probe and the assayed sequence. The probe, in some embodiments, may be attached to a solid support or to a detectable label. The probe will generally be single stranded and will generally be between 10 and 100 nucleotides. The particular properties of a probe will depend upon the particular use and are readily within the competence of one of ordinary skill in the art to determine.

**"*Primer pair*"*-*** a set of two nucleic acid molecules ("primers"), each of which can serve to prime template-directed polymerization by a polymerase or transcriptase, which primers hybridize to the opposite strands of a double stranded nucleic acid sequence ("template") in such manner as to direct the polymerization (and amplification) of the double-stranded sequence nucleotide sequence located between regions of primer hybridization. Such a primer pair can be used in the well known polymerase chain reaction (PCR). The design of primers pairs is well known in the art and will depend upon the particular sequence to be amplified. In general, the primers are single-stranded, between 10 and 40 bases in length and hybridize to regions of the template sequence located between 50 and 2000 bases apart.

**"*Original nucleic acid sequence*"** *-* the nucleic acid sequence of the CD44 transcript present in synovial cells of non-rheumatoid arthritis individuals, having exons 1-5, 7-17 and 19, with splice junctions as described in Screaton et al. (1992).

**"*Original protein sequence*"***-* the amino acid sequence of the CD44 protein encoded by the original nucleic acid sequence.

### GENERAL DESCRIPTION OF THE INVENTION

In accordance with the present invention, it has been found that synovial cells removed from the joints of rheumatoid arthritis (RA) patients express a variant mRNA transcript of CD44 that is not found in synovial cells from healthy (that is, non-RA) individuals. This variant CD44 transcript has not previously been described. This CD44 variant will be referred to herein interchangeably as *"RA-CD44 variant nucleic acid coding sequence* " or "*variant coding sequence* " or *"RA-CD44".* In accordance with the invention, it was found that the novel RA-CD44 mRNA transcript which contains the constant Exons 1-5, 15-17 and 19 and variant Exons 7-14 (v3-v10) (as described by Screaton *et al.,* 1992) also comprises three additional nucleotides (CAG) that are transcribed from the end of the intron bridging Exon v4 to Exon v5 and inserted at the 5' end of Exon v5 in positions 908-910 of SEQ ID NO:1. This extra CAG sequence results in an insertion of a new codon for the amino acid alanine. The last 3' original nucleotide "*G*" of Exon v4 (in position 907 of SEQ ID NO: 1) together with the new "*CA*" nucleotides at the 5' end of the Exon v5 (positions 908 and 909 of SEQ ID NO:1) form an additional codon "*GCA* " which encodes the amino acid alanine (in position 303 of SEQ ID NO:2). The translation at both sides of the new insert is not changed as the original "*GAT*" codon (which encodes the amino acid aspartic acid) is preserved (by the new nucleotide "*G*" in position 910 of SEQ ID NO: 1 and the next two original nucleotides " AT" in positions 911 and 912 of SEQ ID NO:1) as well as all the other codons of Exon v4 and v5. The generation of the extra CAG in the CD44 transcript of RA patient's synoviocytes is shown below:

To date, the novel CD44 mRNA comprising the CAG insertion was detected in synovial cells of eighteen out of eighteen RA patients in which the V₃-V₁₀ CD44 isoform was detected. The sequence of this novel transcript is presented in SEQ ID NO:1.

The above findings of a novel RA-CD44 coding variant open the way for diagnosis, prognosis, prevention and treatment of RA and other diseases in which the variant CD44 of the invention is involved. In addition, the novel RA-CD44 variant may be useful for diagnosis, prognosis, prevention and treatment of other disorders and diseases which involve cells which express other forms of the CD44 protein.

The novel RA-CD44 variant of the invention is a naturally occurring sequence which has not been detected in cells of healthy individuals but only in those of individuals suffering from rheumatoid arthritis. The RA-CD44 variant is presumably produced by alternative splicing of the primary transcript of the known CD44 gene which occurs in cells of such patients and does not arise from truncation or mutation of the known CD44 gene.

The present invention provides by a first aspect, a novel isolated nucleic acid molecule being an alternative splice variant and encoding a CD44 variant and which comprises the three additional nucleotides CAG resulting in an insertion of a codon for the amino acid alanine at the 5'-end of exon v5 of the CD44 gene selected from the group consisting of:
(a) a nucleic acid molecule comprising or consisting of the coding sequence SEQ ID NO: 1; and
(b) a nucleic acid molecule having at least 90% identity to the sequence of (a), wherein said nucleic acid molecule comprises the three additional nucleotides CAG in a region corresponding to the region of nucleotides 908-910 of SEQ ID NO: 1.

The present invention also provides fragments of (a) having at least 20 nucleotides, wherein said fragment comprises the three additional nucleotides CAG in a region corresponding to the region of nucleotides 908-910 in SEQ ID NO: 1. All of the above nucleic acid molecules will retain the nucleotide region corresponding to nucleotides 908-910 of SEQ ID NO:1. Nucleic acid molecules having a sequence complementary to any of the above are also included.

The present invention further provides a polypeptide comprising or consisting of the amino acid sequence of SEQ ID NO:2 and fragments comprising at least 6 amino acids of the sequence of SEQ ID NO:2, homologues which are at least 90% identical to the sequence of SEQ ID NO:2, or at least 90% identical to fragments of at least 6 amino acids thereof, and fragments of the variant product, and its homologues, having at least 10 amino acids. All of the above polypeptides, fragments and homologues will retain the amino acid residue corresponding to residue 303 of SEQ ID NO:2.

Due to the fact that the region which differs in the variant of the invention as compared to the original sequence is in the 5' part of exon v5, the homologues of the variant which are within the scope of the invention should be such which are derivated from the variant by a deletion, addition or substitution of an amino acid either in said region or in regions adjacent to it, but excluding deletion or substitution of the additional alanine residue.

Due to the degenerate nature of the genetic code, a plurality of alternative nucleic acid sequences other than that depicted in SEQ ID NO: 1 can code for the polypeptide of the invention. Thus, the present invention further provides nucleic acid molecules comprising or consisting of a sequence which encodes the polypeptides of the invention. Alternative nucleic acid sequences coding for the same amino acid sequences coded by the sequence of SEQ ID NO: 1 are also an aspect of the present invention.

The present invention additionally provides a fusion protein comprising the polypeptide having the sequence of SEQ ID NO:2, or fragments or homologues thereof, fused to another protein or peptide, as is well known in the art.

The present invention further provides expression vectors and cloning vectors comprising any of the above nucleic acid molecules, as well as host cells transfected by said vectors.

In view of the fact that the polypeptide of the invention contains a unique additional region which does not appear in CD44 obtained from synoviocytes from healthy individuals, antibodies specifically directed against the RA-CD44 variant product, may be used specifically for diagnosis, prognosis, prevention and therapy of rheumatoid arthritis. By "specifically directed against" or "specifically bind" the RA-CD44 variant product is meant that the antibody recognizes and binds to the RA-CD44 variant product in preference to other known CD44 proteins; in particular the anti-RA-CD44 antibodies will recognize and bind to the RA-CD44 variant product in preference to the original protein sequence.

Thus, by an additional aspect, the present invention provides anti-RA-CD44 antibodies selected from the group consisting of:
(a) antibodies which specifically bind the polypeptide encoded by the RA-CD44 variant nucleic acid coding sequence;
(b) fragments of the antibodies of (a) substantially retaining the antigen binding characteristics of the whole antibody; and
(c) antibodies binding to an antigenic epitope bound by any one of the antibodies of (a) and (b) above.

The antibodies of the invention can be polyclonal antibodies or monoclonal. By a preferred embodiment, the antibodies of the invention are such which specifically bind the polypeptide of SEQ ID NO: 2. Fragments of such antibodies substantially retaining the antigen-binding characteristics of these antibodies, antibodies binding to an antigenic epitope bound by such antibodies, as well as antibodies which bind to an antigen to which any one of the above Abs specifically bind are also within the scope of the invention. The anti-RA-CD44 antibody of the present invention will recognize an epitope of the RA-CD44 variant protein that is not present in the original protein sequence. The anti-RA-CD44 antibodies of the invention will preferably recognize an epitope comprising the alanine at residue 303 of SEQ ID NO:2. Alternatively, or in addition, the anti-RA-CD44 antibodies of the invention will recognize a neoepitope created by a change in the overall tertiary structure of the CD44 protein as a consequence of the alanine resudue insertion at position 303.

In accordance with this embodiment, the antibodies of the invention are used for diagnosis or treatment of RA.

The present invention further provides mouse hybridoma cell lines which produce any of the monoclonal Abs of the invention. The hybridomas may be prepared by any of the methods known in the art (e.g. Kohler, G. and Milstein, C., Nature, 256:495-497, (1975). The present invention further provides recombinant cell lines or transgenic animals expressing human or humanized anti-RA-CD44 antibodies of the invention.

By yet an additional aspect, the present invention provides a nucleic acid molecule comprising or consisting of a non-coding sequence which is complementary to that of SEQ ID NO: 1 or complementary to a sequence having at least 90% identity to said sequence (under the conditions defined above) or a fragment of said two sequences (according to the above definition of fragment). The complementary sequence may be a DNA sequence which hybridizes with the sequence of SEQ of ID NO: 1or hybridizes to a portion of that sequence having a length sufficient to inhibit the transcription of the complementary sequence. The complementary sequence may be a DNA sequence which can be transcribed into an mRNA being an antisense to the mRNA transcribed from SEQ ID NO: 1 or into an mRNA being an antisense to the mRNA transcribed from SEQ ID NO: 1 or into an mRNA which is an antisense to a fragment of the mRNA transcribed from SEQ ID NO: 1 which has a length sufficient to hybridize with the mRNA transcribed from SEQ ID NO: 1. so as to inhibit its translation. The complementary sequence may also be the mRNA or the fragment of the mRNA itself. Such complementary sequences may be used for various diagnostic and therapeutic indications as explained below.

In accordance with the therapeutic aspect of the invention, the invention also provides pharmaceutical compositions comprising a pharmaceutically acceptable carrier and, as an active ingredient, RA-CD44 variant coding sequence, expression vectors and cloning vectors comprising said coding sequence, and host cells transfected by said vector.

The above pharmaceutical compositions may be used for the prevention or treatment of diseases and disorders which can be ameliorated or cured by raising the in vivo level of the variant product of the invention. Such diseases or disorders may be infectious and other inflammatory diseases and autoimmune diseases, malignant diseases or any other diseases which involve cells which express the CD44 protein. By a preferred embodiment, these pharmaceutical compositions are used for the prevention or treatment of disorders or diseases which involve cells which comprise or express the RA-CD44 variant. Most preferably, said pharmaceutical compositions are used for the prevention or treatment of RA.

In addition, in accordance with this aspect of the invention, the invention further provides pharmaceutical compositions comprising a pharmaceutically acceptable carrier and, as an active ingredient, an anti-RA-CD44 antibody; including, an antibody which specifically binds to the RA-CD44 variant product, fragments of said antibody substantially retaining the antigen-binding characteristics of the whole antibody and an antibody binding to an antigenic epitope bound by said antibody.

Furthermore, the invention also provides pharmaceutical compositions comprising a pharmaceutically acceptable carrier and, as an active ingredient, a nucleic acid molecule comprising or consisting of a non-coding sequence which is complementary to the variant coding sequence or to a sequence having at least 90% identity to said sequence; a fragment of said sequences, expression vectors comprising any one of said complementary sequences and host cells transfected with said complementary sequences or vectors comprising the sequences, together with a pharmaceutically acceptable carrier.

The above pharmaceutical compositions comprising said anti-RA-CD44 antibodies or the nucleic acid molecule comprising said complementary sequence, are suitable for the prevention or treatment of diseases and disorders where a therapeutically beneficial effect may be achieved by neutralizing the RA-CD44 variant (either at the transcript or product level) or decreasing the amount of the variant product or blocking its binding to its target, for example, by the neutralizing effect of the antibodies, or by the decrease of the effect of the mRNA in decreasing expression level of the variant product with antisense. Such diseases or disorders may, for example, be infectious and other inflammatory diseases and autoimmune diseases, malignant diseases or any other disease or disorder in which cells comprising the RA-CD44 variant or expressing the RA-CD44 protein are involved. In addition, such pharmaceutical compositions may be used for the treatment of diseases which involve cells expressing other forms of the CD44 protein.

In accordance with a preferred embodiment, the pharmaceutical composition comprising said anti-RA-CD44 antibodies or said nucleic acid molecule comprising said complementary sequence are used for the treatment of autoimmune diseases. Most preferably, said pharmaceutical compositions are used in the treatment of RA.

In accordance with the diagnostic aspect of the invention, methods for identifying an individual having rheumatoid arthritis is provided. The method is based on detecting the presence or level of the transcript RNA of the RA-CD44 variant product in a body fluid sample, preferably a synovial cell sample, obtained from the tested individual. The detection of the presence of the mRNA of the variant product may be indicative of RA in a tested individual from which the sample was obtained.

According to this aspect, a method is provided for identifying an individual having rheumatoid arthritis comprising the steps of:
(a) obtaining a biological sample from said tested individual;
(b) providing a probe comprising at least one of the RA-CD44 nucleic acid molecules of the invention;
(c) contacting said biological sample with said probe under conditions allowing hybridization of said probe to said RA-CD44 variant coding sequence transcript and the formation of detectable probe-transcript hybridization complexes;
(d) detecting said probe-transcript hybridization complexes, wherein the presence of said complexes indicates a high probability that the tested individual has RA.

The biological sample used in the above method can be any appropriate biological sample including but not limited to whole blood, peripheral blood monocytes, leukocytes, etc. In a preferred embodiment, the biological sample will comprise synoviocytes or synovial fluid, or RNA (either total RNA or polyA RNA) isolated from the same. Detection of the probe-variant hybridization complexes can be carried out by any of a number of techniques well known in the art, including, without limitation those described in Sambrook et al. (1989).

The above method which is described as a qualitative one, may also be quantitative. In accordance with such a quantitative method, the level of hybridization complexes in healthy individuals as well as the level of the complexes formed in individuals suffering from rheumatoid arthritis is first determined. The level of the complexes detected in the tested individual is then compared to the known calibrated levels of the transcripts.

The probe used in the above method may be DNA or RNA , etc; it may be a coding sequence or a sequence complementary thereto (for respective detection of RNA transcripts or coding-DNA sequences). By quantization of the level of hybridization complexes and calibrating the quantified results it is possible also to detect the level of the transcript in the sample.

According to this same aspect, another method is provided for identifying an individual having rheumatoid arthritis, which method comprises the steps of:
(a) obtaining a biological sample from said tested individual;
(b) providing a primer pair capable of priming the amplification of a region of the RA-CD14 variant coding sequence transcript;
(c) contacting said biological sample with said primer pair under conditions allowing amplification of said RA-CD44 variant coding sequence transcript and the formation of detectable amplification product;
(d) detecting said amplification product, wherein the presence of said amplification product indicates a high probability that the tested individual has RA.

Amplification of the RA-CD44. variant coding sequence transcript can conveniently be accomplished using the well known polymerase chain reaction (PCR) technique (Saiki et al. Science 230: 1350(1995); Mullis et al. Methods Enzymol.155:335 (1987); Erlich et al. Nature (London) 331:461 (1988)). A primer pair is chosen to amplify an appropriate portion of the RA-CD44 variant coding sequence transcript as is well within the competence of one of ordinary skill in the art. The primer pair will typically amplify a portion of the RA-CD44 variant coding sequence transcript in the region in which the variant transcript differs from the original nucleic acid sequence.

By a preferred embodiment, the above diagnostic or prognostic methods are used for diagnosis or prognosis of RA or for following up the disease in a tested individual. According to this embodiment, the nucleic acid probe contacted with the sample is such which is able to specifically detect the presence of an mRNA transcribed from the variant coding sequence having the sequence of SEQ ID NO: 1,or the primer pair is such as to amplify a portion of the mRNA transcribed from the variant coding sequence having the sequence of SEQ ID NO: 1. In particular, the nucleic acid probe or the primer pair will be such as to allow detection of the presence of an mRNA transcribed from the variant coding sequence having the sequence of SEQ ID NO: 1, in preference to detection of an mRNA transcript having the original nucleic acid sequence. In accordance with the findings of the present invention, a qualitative method may be sufficient to identify an individual suffering from RA since the novel coding sequence has been identified in such patents only. However, various degrees of the disease as well as other related diseases or disorders may be identified using a quanitative method as described above.

In accordance with an additional diagnostic aspect of the invention, a method for identifying an individual having a high probability of having rheumatoid arthritis is based on the detection of the variant product in a sample obtained from said individual is also provided comprising the steps of:
(One) obtaining a biological sample from said tested individual;
(Two) contacting said biological sample with an anti-RA-CD44 antibody of the invention under conditions enabling the formation of a detectable antibody-antigen complex; and
(c) detecting said antibody-antigen complex, the presence of said antibody-antigen complex indicating a high probability that the tested individual has RA.

The biological sample used in the above method can be any appropriate biological sample including whole blood, peripheral blood monocytes, leukocytes, etc., preferably the biological sample will comprise synoviocytes or synovial fluid, or cellular extracts thereof. Detection of the antibody-antigen complexes can be carried out by any of a number of techniques well known in the art, including, without limitation those described in Harlow and Lane, Antibodies: A Laboratory Manual, Cold Spring Harbor Laboratory, 1988.

As described with regards to the diagnostic assays based on the detection of the coding sequence mRNA transcript, in this case as well, the method may be quantitized to determine the level or amount of the variant product in the sample which may be indicative of rheumatoid arthritis or the extent of RA from which the tested individual is suffering.

As indicated above, this method as well can be quantitized to determine the level or the amount of the RA-CD44 variant product in the sample, alone or in comparison to the level of the original protein sequence.

By yet another aspect the invention also provides a method for identifying candidate agonist or antagonist compounds of the RA-CD44 variant product comprisng:
(a) providing a polypeptide comprising an amino acid sequence substantially as depicted in SEQ ID NO:2;
(b) contacting a tested candidate compound with said polypeptide;
(c) measuring the effect of said candidate compound on the activity of said polypeptide and selecting those compounds which show at least 70%, preferably 90%, reduction of the level or duration of said activity (antgonists) or at least 70%, preferably 90%, increase in the level or duration of said activity (agonists). Any assay measuring a known activity of CD44 may be used to test the effect of the candidate compound such as for example, cell adhesion, rolling, extravasation or migration of cells.

Wherein the candidate agonist is one that has an activity which is essentially identical to the activity of RA-CD44, the method for identifying it will comprise the following steps:
(a) providing a polypeptide comprising an amino acid sequence substantially as depicted in SEQ ID NO:2;
(b) measuring the activity of said polypeptide in a test assay;
(c) measuring the activity of said candidate compound in said test assay; and
(d) comparing the activity measured in (c) to the activity measured in (b),
   wherein an activity measured in (c) being at least 70%, preferably 90%, of the activity measured in (b) indicating that said candidate compound is a compound having an activity which is essentially identical to the activity of the RA-CD44 variant product.

The test assay in the above methods may be any assay in which the activity of RA-CD44 may be measured such as for example, the assays measuring the activities mentioned above.

Accordingly, herein described are compounds identified by the above methods.

In accordance with yet another aspect of the invention, peptide ligands which bind to the RA-CD44 variant product of the invention are identified. The identification of such ligands may be carried out, for example, by using phage display peptide libraries. Methods involving use of such libraries are described, for example in Nissim A., EMBO J., 13:692 (1994). The phage libraries used may be antibody libraries as well as peptide libraries.

Thus a method is provided for the identification of a peptide which binds to the RA-CD44 variant product comprising:
(i) incubating cells expressing the RA-CD44 variant product with a phage display peptide library;
(ii) washing the cells to remove unbound phages;
(iii) eluting bound phage from the cells;
(iv) amplifying the resulting bound phage;
(v) determining the display peptide sequence of the bound phage.

The peptides isolated by the above method comprise an additional aspect of the present invention. The isolated peptides are synthesized by any of the methods known in the art including chemical (organic) synthesis, recombinant technologies, computer-aided design technologies, etc. The peptide ligands obtained by the above methods may be used for various diagnostic, prognostic and therapeutic applications. Such ligands may also be used as agonists or antagonists of the variant product.

In view of the finding of the present invention, herein disclosed are uses of a therapeutically effective amount of the RA-CD44 variant nucleic acid coding sequence, or the complementary sequence, or of the anti-RA-CD44 antibodies of the invention in methods for the prevention or treatment of disorders and diseases in an individual in which a therapeutically beneficial effect may be achieved by inhibiting or preventing the expression of the RA-CD44 variant product in cells of the treated individual. These methods can be used for the treatment of infectious and other inflammatory diseases and autoimmune diseases or malignant diseases. Most preferably, the methods are used for the prevention or treatment of RA. Furthermore described herein is the use of a therapeutically effective amount of the RA-CD44 variant coding sequence, vectors comprising it or host cells comprising it in methods for the prevention or treatment of diseases in an individual where there is a therapeutically beneficial effect in raising the level of the RA-CD44 variant product in cells of the individual.

In addition, the invention also encompasses within its scope the
anti-RD-CD44 antibodies of the invention for use in the prevention or treatment of RA.

### DETAILED DESCRIPTION OF ASPECTS OF THE INVENTION

### A. RA-CD44 Nucleic Acid Molecule

The RA-CD44 nucleic acid molecule of the invention includes nucleic acid molecules comprising the RA-CD44 variant nucleic acid coding sequence of the invention including nucleic acid molecules having the sequence shown in SEQ ID NO: 1, nucleic acid molecules having at least 90% identity to said sequence (as described above) and fragments of the above molecules of least 20 nucleotides (as described above), wherein said nucleic acid molecule comprises the three additional nucleotides CAG in a region corresponding to the region of nucleotides 908-910 in SEQ ID NO: 1. The RA-CD44 nucleic acid molecule of the invention additionally includes nucleic acid molecules that are complementary to any of the above mentioned. The nucleic acid molecule may be in the form of DNA or in the form of RNA and include DNA, cDNA and genomic DNA and mRNA, synthetic DNA or RNA. The DNA may be doubled stranded or single stranded and, if single stranded may be the coding strand or the non-coding strand.

The RA-CD44 nucleic acid molecule may include the RA-CD44 variant coding sequence only or, alternatively, the coding region may be in combination with additional coding sequences such as those coding for fusion protein or signal peptides. In addition it may be combined with non-coding sequences such as introns and control elements.

Fragments as defined above are also within the scope of the present invention. Such fragments, typically comprise at least 20 bases which correspond to a region of the variant coding sequence. Preferably, the fragment comprises at least 30, 40, 50, 60, 70 80, 90, or 100, or more, nucleotides which correspond to a region of the variant coding sequence.

The novel RA-CD44 transcript discovered by the present inventors differs from the previously described CD44 transcript by a variation of the splice site junction between exon 8 and exon 9 (Screaton et al., supra). This splice site variation results in the insertion of three additional nucleotides, "CAG" in the sense strand, at the splice junction between exons 8 and 9. Accordingly, the nucleic acid molecule of the invention, whether comprising the sequence of SEQ ID NO: 1, or a complement or a fragment thereof, or sequences that are at least 90% identical to the sequence of SEQ ID NO:1; or a complement or fragment of thereof, will comprise at a minimum, the three nucleotides corresponding to the variant splice junction site as is explained herein above.

By a preferred embodiment, the RA-CD44 nucleic acid molecule comprises the sequence of SEQ ID NO:1 or fragments thereof or sequences having at least 90% identity to the above sequence as explained above. In addition, the RA-CD44 nucleic acid molecule includes nucleic acid sequence coding for the polypeptide of SEQ ID NO:2 or for fragments or homologues of said polypeptide as explained above. The coding sequence may be obtained by any of the methods known in the art. Typically screening of cDNA libraries using oligonucleotide probes which can hybridize to or PCR-amplify nucleic acid sequences which encode the variant products of the invention. A variety of cDNA libraries are commercially available and the procedures for screening and isolating cDNA clones are within the scope of a person skilled in the art. Such techniques are described in, for example, Sambrook et al., (1989) Molecular Cloning: A Laboratory Manual (2nd Edition), Cold Spring Harbor Press, Plain View, New York. Preferably, the cDNA library is prepared from synovial cells.

The nucleic acid molecules can also be synthetically prepared in accordance with chemical synthesis methods known in the art. Alternatively, the nucleic acid molecules can be prepared recombinantly.

In addition, the variant nucleic acid molecule of the invention may be prepared by extracting cellular RNA from cells expressing the variant product. Such cells may, for example, be synovial cells of RA patients. The RNA is then subjected to reverse transcriptase polymerase chain reaction (RT-PCR) in accordance with methods known in the art such as those described in Sambrook *et al.. supra.* The amplification products of the PCR reaction are purified and sequenced.

### B. Comparison of the Variant Coding Sequence to the known CD44 Sequences

The complete known sequence of the CD44 gene has been published and can be found for example in Screaton *et al.,* 1992, *supra,* which also describes the location of exon-intron junctions. Comparison of the coding sequence of the invention to the known CD44 sequence may be carried out by any of the available computer programs.

### III. Expression Vectors, Cloning Vectors and Host Cells

The present invention also includes recombinant constructs comprising one or more of the RA-CD44 variant nucleic acid molecules described above. The constructs comprise a vector, such as a plasmid or viral vector, into which a nucleic acid molecule of the invention has been inserted, in a forward or reverse orientation. In a preferred aspect of this embodiment, the construct further comprises regulatory sequences, including, for example, a promoter, operably linked to the RA-CD44 sequence. Large numbers of suitable vectors and promoters are known to those of skill in the art, and are commercially available. Appropriate cloning and expression vectors for use with prokaryotic and eukaryotic hosts are also described in Sambrook, *et al., (supra).*

The present invention also relates to host cells which comprise vectors of the invention as well as to the production of the RA-CD44 variant product of the invention by recombinant techniques. Host cells are genetically engineered (i.e., transduced, transformed or transfected) with the vectors of this invention which may be, for example, a cloning vector or an expression vector. The vector may be, for example, in the form of a plasmid, a viral particle, a phage, etc. The engineered host cells can be cultured in conventional nutrient media modified as appropriate for activating promoters, selecting transformants or amplifying the expression of the variant nucleic acid sequence. The culture conditions, such as temperature, pH and the like, are those previously used with the host cell selected for expression, and will be apparent to those skilled in the art.

The RA-CD44 variant nucleic acid coding sequences of the present invention may be included in any one of a variety of expression vectors for expressing a product. Such vectors include chromosomal, nonchromosomal and synthetic DNA sequences, e.g., derivatives of SV40; bacterial plasmids; phage DNA; baculovirus; yeast plasmids; vectors derived from combinations of plasmids and phage DNA, viral DNA such as vaccinia, adenovirus, fowl pox virus, and pseudorabies. However, any other vector may be used as long as it is replicable and viable in the host. The appropriate RA-CD44 variant DNA sequence may be inserted into the vector by a variety of procedures. In general, the DNA sequence is inserted into an appropriate restriction endonuclease site(s) by procedures known in the art. Such procedures and related sub-cloning procedures are deemed to be within the scope of those skilled in the art.

The RA-CD44 variant coding sequence in the expression vector is operatively linked to an appropriate transcription control sequence (promoter) to direct mRNA synthesis. Examples of such promoters include: LTR or SV40 promoter, the *E.coli lac* or *trp* promoter, the phage lambda *PL* promoter, and other promoters known to control expression of genes in prokaryotic or eukaryotic cells or their viruses. The expression vector also contains a ribosome binding site for translation initiation, and a transcription terminator. The vector may also include appropriate sequences for amplifying expression. In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in *E.coli.*

The vector containing the appropriate RA-CD44 variant DNA sequence as described above, as well as an appropriate promoter or control sequence, may be employed to transform an appropriate host to permit the host to express the RA-CD44 variant protein. Examples of appropriate expression hosts include: bacterial cells, such as *E.coli, Streptomyces, Salmonella typhimurium;* fungal cells, such as yeast; insect cells such as *Drosophila* and *Spodoptera* Sf9; animal cells such as CHO, COS, HEK 293 or Bowes melanoma; adenoviruses; plant cells, etc. The selection of an appropriate host is deemed to be within the scope of those skilled in the art from the teachings herein. The invention is not limited by the host cells employed.

In a further embodiment, the present invention relates to host cells containing the above-described constructs. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation (Davis, L., Dibner, M., and Battey, I. (1986) Basic Methods in Molecular Biology). Cell-free translation systems can also be employed to produce polypeptides using RNAs derived from the DNA constructs of the present invention.

The variant products can be recovered and purified from recombinant cell cultures by any of a number of methods well known in the art, including ammonium sulfate or ethanol precipitation, acid extraction, anion or cation exchange chromatography, phosphocellulose chromatography, hydrophobic interaction chromatography, affinity chromatography, hydroxylapatite chromatography, and lectin chromatography. Protein refolding steps can be used, as necessary, in completing configuration of the mature protein. Finally, high performance liquid chromatography (HPLC) can be employed for final purification steps.

### IV. Antibodies and Hybridomas

The anti-RA-CD44 antibodies of the invention include (a) antibodies which specifically bind the polypeptide encoded by the RA-CD44 variant nucleic acid coding sequence, particularly, antibodies which specifically bind the polypeptide having the amino acid sequence of SEQ ID NO:2, (b) fragments of the antibodies of (a) which substantially retain the antigen binding characteristics of the whole antibody: and (c) antibodies which specifically bind to an antigenic epitope bound by any one of the antibodies of (a) and (b) above. The antibodies may be of any of the classes IgG, IgM, IgD, IgA and IgE. The antibodies can be polyclonal or monoclonal. Although, typically, the anti-RA-CD44 antibodies of the invention are produced by hybridoma cell lines as described above, they may also be produced by recombinant genetic methods well known to a person skilled in the art. The antibody may be animal derived, typically a mouse antibody as well as a human antibody, a chimeric antibody, a "*humanized antibody*"*,* a primatized antibody, etc.

Fragments of the antibodies which fall under the scope of the present invention are such which substantially retain the Ag binding characteristics of the whole antibodies from which they are derived and thus specifically bind to the variant product of the invention or to cells or tissues expressing the variant product. The term "*substantially retain*" should be understood to mean that the binding affinity of the antibody fragment for the variant product as determined by any of the methods mentioned below is at least 50% of the binding affinity of the whole antibody for the same variant product.

Hybridoma cell lines which produce the mAb of the invention are also within its scope. Such hybridomas may be prepared by any of the methods known in the art such as described in Kohler, G., *et al., supra.* The supernatant of the hybridoma cell lines are typically screened for antibody binding activity by any one of the methods known in the art such as by enzyme linked immuno sorbent assay (ELISA) or radio immuno assay (RIA). The supernatants are screened for production of mAbs capable of binding to any of the RA-CD44 variant polypeptides of the invention or to cells expressing said polypeptides.

Various hosts can be used for production of antibodies by the hybridoma technique including rats, mice, etc. The animals may be immunized by injecting the variant product or a portion or fragment thereof which retains the immunogenic or antigenic properties of the variant product to the host. Various adjuvants may be used to increase the immunological response such as freund's mineral gels aluminum hydroxide, etc.

In addition to the hybridoma technique mentioned above, continuous cell lines which produce antibodies obtained by additional techniques may also be used such as, for example, the EBV-hybridoma technique (Cole et al., Mol. Cell Biol., 62:109, 1984).

Additionally, wherein the antibodies are recombinantly produced, techniques developed for production of chimeric antibodies or humanized antibodies such as those described in Morrison et al., Proc. Natl. Acad. Sci., 81:6851, (1984) may also be used.

Antibodies may also be produced by inducing *in vivo* production in the appropriates lymphocyte population or by screening recombinant immunoglobulin libraries in accordance with known methods which are described, for example, in Orlandi et al. Post. Natal Acad. Sci. 86:3833, (1989).

Abs may also be produced by DNA immunization with plasmids containing the RA-CD44 cDNA of the invention. Such DNA immunizing methods are described, for example, in Annu. Rev. Immunol., 15:617, (1997).

### V. Variant Product

The RA-CD44 variant product of the invention is a polypeptide encoded by the RA-CD44 variant coding sequence of the invention. The polypeptide has the amino acid sequence of the SEQ ID NO:2, or a fragment of at least 6 amino acids thereof, or a sequence homologue having at least 90% identity to the sequence of SEQ ID NO:2, or at least 90% identity to a fragment of at least 6 amino acids of SEQ ID NO:2, provided that the amino acid sequence is not identical to that of the original protein sequence from which it has been varied. The polypeptides, fragments and homologues of the invention will retain the alanine residue corresponding to amino acid 303 of SEQ ID NO:2 as explained above.

The polypeptide of the invention can be made by any appropriate method including chemical or recombinant synthesis by techniques well known in the art. Fragments may be obtained by enzymatic digestion (e.g. using clostrapaine) or chemical (CNBr) digestion of a longer protein. In such a case, the resulting peptides may be separated by methods known in the art such as by RP-HPLC and the separate peptides may then be used for sequencing (e.g. by Euro sequence BV).

The polypeptides in accordance with the invention may also be synthesized by methods known in the art such as on Abbymed 522 by Euro sequence BV

The variant products may also be obtained by recombinant methods known in the art using the variant coding sequence.

### VI. Diagnostic Applications

The RA-CD44 variant coding sequence of the invention may be used to detect and quantitate the expression of the variant mRNA coding for the variant product. Typically, total mRNA is obtained from the sample and contacted with the nucleic acid probe. The probe is hybridized with the mRNA and the presence of a hybridization product indicates the presence of the variant coding sequence in the sample.

In an alternative diagnostic method, a polymerase chain reaction, or other similar DNA/RNA amplification technique, can be used to detect the presence of a variant RA-CD44 transcript in a sample. In this method a pair of primers is used to amplify the region of the variant RA-CD44 transcript at the variant splice junction. In accordance with techniques well known in the art, a primer may comprise the sequence of the variant splice junction itself, or its complementary sequence , or one or both primers may comprise sequence adjacent to the variant splice junction site or the complementary sequence. One of skill in the art, provided with the description of the RA-CD44 variant transcript and the disclosure of the variant splice site junction, will be competent to select a primer pair for amplification of a region of the RA-CD44 variant transcript that will be diagnostic for its presence, i.e., will distinguish the presence of the RA-CD44 variant transcript from the normal CD44 transcript.

The novel RA-CD44 variant peptide is also useful, as a diagnostic agent for RA by detecting its presence in a sample obtained from a tested individual. Such a sample may be any sample taken from the individual which may contain cells expressing the RA-CD44 variant protein. Such a sample may, for example, be a biopsy specimen, tissues, cells or body fluid comprising such cells.

The anti-RA-CD44 antibodies of the invention may also be used for diagnostic applications. Such antibodies which specifically bind to the RA-CD44 variant product are useful for the diagnosis of conditions or diseases characterized by expression of the RA-CD44 variant product of the invention. Alternatively, such antibodies may be used in assays to monitor patients being treated with the nucleic acid molecule of the invention, the polypeptide of the invention, the variant product, its agonists, or its antagonists.

A variety of protocols which are useful for assaying the variant product, using either polyclonal or monoclonal antibodies, are known in the art. Examples include enzyme-linked immunosorbent assay (ELISA), radioimmunoassay (RIA), and fluorescent activated cell sorting (FACS). Direct or competitive binding assays may also be used.

Diagnostic assays for variant product include methods utilizing the anti-RA-CD44 antibody and a label to detect variant product in human body fluids or extracts of cells or tissues. The variant products and antibodies of the present invention may be used with or without modification. Frequently, the variant proteins and antibodies will be labeled by joining them, either covalently or noncovalently, with a reporter molecule. Such a reporter molecule may for example be a radioactive agent, a fluorescent agent (e.g. FITC) etc.

The above assays may be used for diagnosing rheumatoid arthritis in which the RA-CD44 variant product is expressed at abnormal levels as compared to its expression in healthy individuals. Wherein the variant product is expressed only in abnormal conditions and not in healthy ones, the detection of its presence is sufficient to diagnose the abnormal condition. In cases wherein the variant product is expressed to some extent also in healthy individuals, normal standard expression values for the variant product may be determined by obtaining body samples from a number of healthy individuals and determining the level of expression of the variant product in a pool of these samples. The measured variable expression of the variant product in the tested individual may then be compared to the previously determined standard level.

### VII. Therapeutic uses

The RA-CD44 variant nucleic acid molecule, the RA-CD44 variant product and the anti-RA-CD44 antibodies of the invention all have therapeutic applications in accordance with the invention.

The RA-CD44 variant nucleic acid molecule of the invention may be provided under the control of suitable control elements and thus may result in alteration of the level of expression of the variant product of the invention. Thus, the level of expression of the variant product may, for example be inhibited by using antisense technology wherein gene expression is controlled through hybridization of complementary nucleic acid sequences, i.e. antisense DNA or RNA to be controlled, 5' or regulatory regions of the gene encoding the variant product.

Compositions which comprise the RA-CD44 variant nucleic acid molecule of the invention together with a pharmaceutically acceptable carrier or excipient may also be used in accordance with the invention. Such carriers may be any of the carriers known in the art such as saline, buffer saline, dextrose, glycerol, etc. which may be chosen taking into consideration the amount of administration of the composition as known in the art.

The variant coding sequence of the invention may also be used wherein the coding sequence being a DNA or RNA molecule is introduced into appropriate cells which may then be administered to an individual in need. Such cells may be produced either *ex vivo* or, alternatively, *in vivo* by administering the coding sequence to an individual in an expression vehicle such as, for example, a retrovirus as known in the art. Immunization with the RA-CD44 variant cDNA may be used as well for active immunization against the RA-CD44 variant product.

It is disclosed herein that the variant product described herein may be used for the treatment of disorders and diseases which involve its expression at levels which differ from the level of expression in normal individuals. The variant product or fragments thereof may be administered to an individual for example, in order to raise the level of antibodies against the RA-CD44 product, eventually resulting in lowering the level of this variant protein in the treated individual. The variant product or fragment thereof of the invention may be administered by any one of a number of administration routes including oral, intravenous, intramuscular, subcutaneous, etc. The variant product may be administered alone or in combination with additional treatments. The appropriate formulations and carriers with which the variant product should be administered to a treated individual can be determined by a person skilled in the art in accordance with the administration mode as well as other criteria known to the artisan.

Apart from being used for the prevention or treatment of RA, the anti-RA-CD44 antibodies of the invention may also be used as therapeutic agents for treatment of disorders or diseases involving the expression of the RA-CD44 product of the invention. Administration of the antibodies of the invention to an individual will result in blocking or decreasing the activity of the RA-CD44 variant protein and treatment of such disorders and diseases in which a beneficial effect can be achieved by such a decrease. The antibodies of the invention will typically be administered within pharmaceutical compositions comprising one or more of the antibodies, preferably monoclonal antibodies, of the invention as active ingredients together with pharmaceutically acceptable carriers. The antibodies may be conjugated to radioactive agents, enzymes, antibiotics or toxic agents and used as carriers bringing the agents (drugs) into target cells comprising the RA-CD44 variant.

Typically, such diseases are infectious and other inflammatory diseases and autoimmune diseases such as RA, malignant diseases as well as others. Treatment of such disorders and diseases will be carried out by administering to an individual in need a therapeutically effective amount of one or more of the antibodies of the invention, a therapeutically effective amount being an amount capable of alleviating the symptoms of the disease, reducing its symptoms or completely eliminating them. In a preferred embodiment, the invention provides a method for the treatment or prevention of rheumatoid arthritis, comprising administering to an individual in need a therapeutically effective amount of one or more of the antibodies of the invention.

The RA-CD44 variant nucleic acid coding sequence of the invention, the RA-CD44 variant product and the anti-RA-CD44 antibodies of the invention may also be included in diagnostic or therapeutic kits which comprise an additional aspect of the invention. Diagnostic kits, for example, would typically include one or more of the Abs of the invention, a conjugate of a specific binding partner for the Abs, a label capable of producing a detectable signal and directions for its use. The therapeutic kit will typically include specific Abs conjugated to radioactive, enzymatic, antibiotic or toxic agents.

### EXAMPLES

### A.

### Example 1 Preparation of mAbs which bind the variant product of the invention

Synoviocytes (joint leukocytes) containing the variantly spliced CD44 transcript are removed from the joints of RA patients and used to produce specific monoclonal antibodies (mAbs). Splenocytes of mice immunized with these leukocytes are fused to myeloma cells and hybridomas producing specific mAbs are selected by testing the ability of their supernatants to bind to synoviocytes of RA patients but not to other types of cells from the same or other patients or to cells of normal (i.e., non-RA) individuals.

### Example 2 Diagnosis of RA in tested individuals

Monoclonal antibodies obtained as described in Example 1 above, are incubated with synoviocytes obtained from tested individuals. The level of binding of the mAbs to the synoviocytes is compared to the level of their binding to cells obtained from healthy individuals, a substantially higher level of binding, indicating a high probability that the tested individual is suffering from RA.

### Example 3 Diagnosis of RA using the coding sequence of the invention

Cellular RNA extracted from synovial cells of RA patients with NP-40 was subjected to the reverse transcriptase polymerase chain reaction (RT-PCR). Reverse transcription was performed with 5 U of SuperScript II reverse transcriptase (GibcoBRL) in 20 µl reaction mixes containing 50 mM Tris-HCI ph 8.3, 50 mM MgCl₂ 10 mM DTT, 5 mM spermidine and 20 U Rnasin (Promega), using 1 µg RNA and 100 ng of oligo d(T)₁₈ (BTG, Rehovot, Israel). The reaction mixes were incubated for 1 h at 41°C. PCRs were performed in a microprocessor-controlled incubator using 5 µl of the RT reactions in a reaction volume of 50 µl containing 50 mM KCI, 1.5 mM MgCl₂, 10 mM Tris-HCI pH 9.0. 250 µM dNTPs, 2.5 U Expand High Fidelity (Boehringer Mannheim) and 40 pmol primers:
Sense: 5'-CGCAAGCTTATGGACAAGTTTTGGTGG -3'
Antisense: 5'-ATAGCGGCCGCGTGTGGGCAGAAGAAAA -3'
30 cycles were carried out for CD44 amplifications, each consisting of 1 min at 94°C, 1 min at 55°C and 2 min at 72°C. Amplification products were purified and sequenced.

The presence of the RA-CD44 coding sequence is detected.

### Example 4 Inhibition of experimental arthritis by the mAbs of the invention

(a) mAbs directed against the variant product of the invention are obtained as described above. The mAbs are administered to SCID mice transplanted with synoviocytes of RA patients (Mima, T., et al., J. Clin. Invest. 96:1746. 1995), or in appropriate cases to mice having a collagen-induced arthritis (CIA) (Nedvetzki. J.. Autoimmunity, 1999 in Press). The ability of the mAbs to inhibit the development and symptoms of the experimental arthritis in the mice is evaluated.
(b) Peptides comprising the variant product of the invention are administered to mice described in (a) above.

The capability of the peptides to inhibit experimental arthritis in these mice is evaluated.

### Example 5 Diagnosis of RA by competitive assays

Competitive assays are carried out wherein peptides comprising the variant product of the invention as well as anti-variant product mAbs are contacted with synoviocytes obtained from tested individuals as well as from healthy individuals. The peptide inhibits the Ab binding.

### Example 6 Identification of ligands of the RA-CD44 variant product using phage display peptide library

Use of the phage display peptide library is in accordance with known methods which are described, for example, in Nissim, A., *EMBO, supra*).

### Antibody library source

A human antibody synthetic library (Nissim-N1 library) is used. The diverse repertoire of rearranged V_{H} genes was built *in vitro* by PCR from a bank of human V_{H} gene segments derived from 49 individual germlines and random nucleotide sequence encoding CDR3 lengths of 4-12 residues. The amplified rearranged V_{H} fragments were cloned with a single unmutated V3 segment derived from the germline IGLV3S1 (isolated from an antibody binding to BSA), as a single chain Vᵥ fragment for phage display. This procedure created a single pot phagemid library of more than 10⁸ different clones and is displayed on the N-terminus of the plII of the M 13 bacteriophage.

### Panning strategy and isolation of peptides

A. Namalwa cells (a Burkitt lymphoma derived lymphocyte) transfected with RA-CD44 variant nucleic acid coding sequence and expressing a RA CD44 variant product (Nam. +RA-CD44) are panned with the single pot phagemid antibody library as follows: for the first round of selection, approximately 5 x 10⁶ Nam.+RA-CD44 cells are washed, blocked with PBS containing 4% skim milk and M13 and panned with 10¹² phagemids from N1 library. After incubation at 4°C for 3 h with slow agitation, cells are washed four times with PBS and the bound phages are eluted from the cells at room temperature for 10 min. with 0.1M glycin pH-2.2. After neutralization, cells are spun, discarded, and the supernatant containing the eluted phages is collected and amplified by infecting *E. coli* bacteria (TG-1 strain) and plating them on ampicillin agar plates. Phagemids are rescued by superinfecting exponentially growing amp^{r} bacteria with helper phage (VSC-M13, Stratagene). This amplified stock is used for four additional rounds of panning on Namalwa cells as negatives and Nam.+RA-CD44, with one amplification step after the second panning and without amplification step after the further rounds.
B. Namalwa/CHO-K1 cells transfected with RA-CD44 cDNA are panned with the single pot phagemid antibody library as described above specifically: For the first round of selection approximately 5 x 10⁶ Nam.+RA-CD44 cells are washed, blocked with PBS containing 4% skim milk and M13 and panned with 10¹² phagemids from N1 library. The bound phages are eluted from the cells and the supernatant containing the eluted phages is collected and divided into two steps: one including amplification step by infecting *E.coli* bacteria, and second step is done without amplification. For the second round of selection approximately 5 x 10⁶ Namalwa/CHO-K1 cells are washed, blocked with PBS containing 4% skim milk and M13 and panned with phagemids eluted from the first selection round. The phagemids which do not bind to the Namalwa/CHO-K1 cells are incubated with Namalwa/CHO-K1 cells transfected with RA-CD44 cDNA respectively. The second round of selection was repeated twice.

The peptides corresponding to the displayed peptides are synthesized. Some of the peptides are synthesized chemically by solid phage synthesis (Merrifield, J., Amer. Chem. Soc., 85:2149-2154 (1963)). Other peptides are synthesized by recombinant technology.

## Claims

1. A nucleic acid molecule encoding a CD44 variant and which comprises the three additional nucleotides CAG resulting in an insertion of a codon for the amino acid alanine at the 5'-end of exon v5 of the CD44 gene, selected from the group consisting of:
(a) a nucleic acid molecule comprising or consisting of the sequence of SEQ ID NO: 1;
(b) a nucleic acid molecule comprising a sequence having at least 90% identity to the sequence of (a), wherein said nucleic acid molecule comprises the three additional nucleotides CAG in a region corresponding to the region of nucleotides 908-910 in SEQ ID NO: 1;
(c) a nucleic acid molecule being complementary to (a) or (b);
(d) a nucleic acid molecule encoding a polypeptide having the amino acid sequence of SEQ ID NO: 2; and
(e) a nucleic acid molecule encoding a polypeptide having an amino acid sequence that is at least 90 % identical to SEQ ID NO:2, wherein said nucleic acid molecule comprises the three additional nucleotides CAG in a region corresponding to the region of nucleotides 908-910 in SEQ ID NO: 1.

2. A fragment of a nucleic acid molecule comprising or consisting of the sequence of SEQ ID NO: 1 having at least 20 nucleotides, wherein said fragment comprises the three additional nucleotides CAG in a region corresponding to the region of nucleotides 908-910 in SEQ ID NO:1.

3. An expression vector comprising the nucleic acid molecule of claim 1 or 2, together with control elements enabling the expression of said nucleic acid sequences in a host cell.

4. The expression vector of claim 3, wherein said control elements enable the expression of said nucleic acid sequence in a bacterial host cell or in a fungal host cell.

5. A host cell comprising the expression vector of claim 3 or 4.

6. A polypeptide encoded by the nucleic acid molecule of claim 1 or 2.

7. A fragment of a polypeptide encoded by a nucleic acid molecule comprising the sequence of SEQ ID NO: 1 having at least 6 amino acids, wherein said nucleic acid molecule comprises the three additional nucleotides CAG in a region corresponding to the region of nucleotides 908-910 in SEQ ID NO: 1 wherein the fragment retains the amino acid residue corresponding to residue 303 of SEQ ID NO.2.

8. An antibody which specifically binds to
(i) an epitope of the polypeptide of claim 6 or 7, said epitope comprising the alanine residue corresponding to position 303 of SEQ ID NO:2; and/or
(ii) a neoepitope of the polypeptide of claim 6 or 7, said neoepitope being created as a consequence of the alanine residue corresponding to position 303 of SEQ ID NO:2.

9. A fragment of the antibody of claim 8, wherein said fragment substantially retains the antigen binding characteristics of the antibody of claim 6.

10. The antibody of claim 8 or 9, wherein said antibody is a monoclonal antibody (mAb).

11. An immortalized cell line producing mAbs of claim 10.

12. An immortalized cell line of claim 11, being a hybridoma cell line.

13. An in vitro method for identifying an individual having rheumatoid arthritis comprising the steps of:
(a) providing a probe comprising at least one of the nucleic acid molecules of claim 1 or 2;
(b) contacting a biological sample with said probe under conditions allowing hybridization of said probe with said transcript to form detectable probe-transcript hybridization complexes;
(c) detecting probe-transcript hybridization complexes, wherein the presence of said complexes indicates a high probability that the tested individual from which the sample was obtained has rheumatoid arthritis.

14. The method of claim 13, wherein said nucleic acid probe comprises a sequence detecting the presence of an mRNA transcribed from the coding sequence of SEQ 1D NO: 1.

15. An in vitro method for identifying an individual having rheumatoid arthritis comprising the steps of :
(a) providing a probe comprising at least one of the nucleic acid molecules of claim 1 or 2;
(b) contacting a biological sample with said probe under conditions allowing hybridization of said probe with said transcript to form detectable probe-transcript hybridization complexes;
(c) detecting prohe-transcript hybridization complexes and comparing the level of said complexes to the level of hybridization complexes detected in a biological sample obtained from a healthy individual, a deviation from the level of the hybridization complexes in said healthy individual, indicating a high probability that the tested individual from which the sample was obtained has rheumatoid arthritis.

16. An in vitro method for identifying an individual having a high probability of having rheumatoid arthritis comprising:
(a) contacting a biological sample with the antibody of claim 8, under conditions enabling the formation of a detectable antibody-antigen complex; and
(b) detecting said antibody-antigen complex, the presence of said antibody-antigen complex indicating a high probability that the tested individual has rheumatoid arthrits.

17. An in vitro method for identifying an individual having a high probability of having rheumatoid arthritis comprising:
(a) contacting a biological sample with the antibody of claim 8, under conditions enabling the formation of a detectable antibody-antigen complex; and
(b) detecting said antibody-antigen complex and comparing the level of said complexe to the level of antibody-antigen complexes detected in samples obtained from a healthy individual, a deviation from said level indicating a high probability that the tested individual has rheumatoid arthrits.

18. A method for the identification of a peptide which binds to the polypeptide of claim 6, comprising:
(a) incubating cells expressing said amino acid sequence with a phage display peptide library;
(b) washing the cells to remove unbound phages;
(c) eluting bound phage from the cells;
(d) amplifying the resulting bound phage;
(e) determining the display peptide sequence of the bound phage.

19. A method for identifying candidate agonist compounds of the polypeptide of claim 6, comprising:
(a) providing a polypeptide comprising an amino acid sequence as depicted in SEQ ID NO: 2;
(b) contacting a tested candidate compound with said polypeptide;
(c) measuring the effect of said candidate compound on the activity of said polypeptide and selecting a compound which shows at least a 70% inducing effect on the level or duration of said activity.

20. A method for identifying candidate antagonist compounds of the polypeptide of claim 6, comprising:
(a) providing a polypeptide comprising an amino acid sequence as depicted in SEQ ID NO: 2;
(b) contacting a tested candidate compound with said polypeptide;
(c) measuring the effect of said candidate compound on the activity of said polypeptide and selecting a compound which shows at least a 70% inhibitory effect on the level or duration of said activity.

21. A method for identifying a candidate compound having an activity which is identical to the activity of the polypeptide of claim 6, comprising:
(a) providing a polypeptide comprising an amino acid sequence as depicted in SEQ ID NO: 2;
(b) measuring the activity of said polypeptide in a test assay;
(c) measuring the activity of said candidate compound in said test assay; and
(d) comparing the activity measured in (c) to the activity measured in (b), wherein an activity measured in (c) being at least 70% of the activity measured in (b) indicating that said candidate compound is a compound having an activity which is identical to the activity of the RA-CD44 variant product.

22. A pharmaceutical composition comprising a pharmaceutically acceptable carrier and, a nucleic acid molecule of claim 1 or 2, an expression vector of claim 3 or 4, a host cell of claim 5, a polypeptide of claim 6, or an antibody of any one of claims 8 to 10.

23. The pharmaceutical composition of claim 22 which is for the prevention and treatment of rheumatoid arthritis.

24. Use of an antibody of any one of claims 8 to 10, for the preparation of a pharmaceutical composition, for the prevention or treatment of rheumatoid arthritis.

## Patentansprüche

1. Nucleinsäuremolekül, das eine CD44-Variante codiert und das die drei zusätzlichen Nucleotide CAG umfasst, was zu einer Einfügung eines Codons für die Aminosäure Alanin am 5'-Ende von Exon v5 des CD44-Gens führt, ausgewählt aus der Gruppe bestehend aus:
(a) einem Nucleinsäuremolekül, das die Sequenz von SEQ ID NO: 1 umfasst oder daraus besteht;
(b) einem Nucleinsäuremolekül, das eine Sequenz umfasst, die mindestens 90 % Identität zu der Sequenz von (a) aufweist, wobei das Nucleinsäuremolekül die drei zusätzlichen Nucleotide CAG in einer Region umfasst, die der Region der Nucleotide 908-910 in SEQ ID NO: 1 entspricht;
(c) einem Nucleinsäuremolekül, das zu (a) oder (b) komplementär ist;
(d) einem Nucleinsäuremolekül, das ein Polypeptid codiert, das die Aminosäuresequenz von SEQ ID NO: 2 aufweist; und
(e) einem Nucleinsäuremolekül, das ein Polypeptid codiert, das eine Aminosäuresequenz aufweist, die zu mindestens 90 % identisch zu SEQ ID NO: 2 ist, wobei das Nucleinsäuremolekül die drei zusätzlichen Nucleotide CAG in einer Region umfasst, die der Region der Nucleotide 908-910 in SEQ ID NO: 1 entspricht.

2. Fragment eines Nucleinsäuremoleküls, das die Sequenz von SEQ ID NO: 1 mit mindestens 20 Nucleotiden umfasst oder daraus besteht, wobei das Fragment die drei zusätzlichen Nucleotide CAG in einer Region umfasst, die der Region der Nucleotide 908-910 in SEQ ID NO: 1 entspricht.

3. Expressionsvektor, der das Nucleinsäuremolekül gemäß Anspruch 1 oder 2 zusammen mit Kontrollelementen umfasst, die die Expression der Nucleinsäuresequenzen in einer Wirtszelle ermöglichen.

4. Expressionsvektor gemäß Anspruch 3, wobei die Kontrollelemente die Expression der Nucleinsäuresequenz in einer bakteriellen Wirtszelle oder einer Pilz-Wirtszelle ermöglichen.

5. Wirtszelle, die den Expressionsvektor gemäß Anspruch 3 oder 4 umfasst.

6. Polypeptid, das vom Nucleinsäuremolekül gemäß Anspruch 1 oder 2 codiert wird.

7. Fragment eines Polypeptids, das von einem Nucleinsäuremolekül codiert wird, das die Sequenz von SEQ ID NO: 1 mit mindestens sechs Aminosäuren umfasst, wobei das Nucleinsäuremolekül die drei zusätzlichen Nucleotide CAG in einer Region umfasst, die der Region der Nucleotide 908-910 in SEQ ID NO: 1 entspricht, wobei das Fragment den Aminosäurerest beibehält, der dem Rest 303 von SEQ ID NO: 2 entspricht.

8. Antikörper, der spezifisch bindet an:
(i) ein Epitop des Polypeptids gemäß Anspruch 6 oder 7, wobei das Epitop den Alanin-Rest umfasst, der Position 303 von SEQ ID NO: 2 entspricht; und/oder
(ii) ein Neoepitop des Polypeptids gemäß Anspruch 6 oder 7, wobei das Neoepitop als Folge des Alanin-Rests gebildet wird, der Position 303 von SEQ ID NO: 2 entspricht.

9. Fragment des Antikörpers gemäß Anspruch 8, wobei das Fragment im Wesentlichen die Antigen-bindenden Eigenschaften des Antikörpers gemäß Anspruch 6 beibehält.

10. Antikörper gemäß Anspruch 8 oder 9, wobei der Antikörper ein monoclonaler Antikörper (mAb) ist.

11. Immortalisierte Zelllinie, die mAbs gemäß Anspruch 10 produziert.

12. Immortalisierte Zelllinie gemäß Anspruch 11, die eine Hybridom-Zelllinie ist.

13. *In vitro*-Verfahren zum Identifizieren eines Individuums, das Gelenkrheumatismus hat, das die Schritte umfasst:
(a) das Bereitstellen einer Sonde, die mindestens eines der Nucleinsäuremoleküle gemäß Anspruch 1 oder 2 umfasst;
(b) das Inkontaktbringen einer biologischen Probe mit der Sonde unter Bedingungen, die die Hybridisierung der Sonde mit dem Transkript erlauben, um nachweisbare Sonde-Transkript-Hybridisierungskomplexe auszubilden;
(c) das Nachweisen von Sonde-Transkript-Hybridisierungskomplexen, wobei die Anwesenheit der Komplexe eine hohe Wahrscheinlichkeit anzeigt, dass das getestete Individuum, von dem die Probe erhalten wurde, Gelenkrheumatismus hat.

14. Verfahren gemäß Anspruch 13, wobei die Nucleinsäuresonde eine Sequenz umfasst, die die Anwesenheit einer mRNA nachweist, die von der codierenden Sequenz von SEQ ID NO: 1 transkribiert wurde.

15. *In vitro*-Verfahren zum Identifizieren eines Individuums, das Gelenkrheumatismus hat, das die Schritte umfasst:
(a) das Bereitstellen einer Sonde, die mindestens eines der Nucleinsäuremoleküle gemäß Anspruch 1 oder 2 umfasst;
(b) das Inkontaktbringen einer biologischen Probe mit der Sonde unter Bedingungen, die die Hybridisierung der Sonde mit dem Transkript erlauben, um nachweisbare Sonde-Transkript-Hybridisierungskomplexe auszubilden;
(c) das Nachweisen von Sonde-Transkript-Hybridisierungskomplexen und das Vergleichen des Spiegels der Komplexe mit dem Spiegel an Hybridisierungskomplexen, die in einer biologischen Probe nachgewiesen wurden, die von einem gesunden Individuum erhalten wurde, wobei eine Abweichung vom Spiegel der Hybridisierungskomplexe in dem gesunden Individuum eine hohe Wahrscheinlichkeit anzeigt, dass das getestete Individuum, von dem die Probe erhalten wurde, Gelenkrheumatismus hat.

16. *In vitro*-Verfahren zum Identifizieren eines Individuums, das eine hohe Wahrscheinlichkeit aufweist, Gelenkrheumatismus zu haben, das umfasst:
(a) das Inkontaktbringen einer biologischen Probe mit dem Antikörper gemäß Anspruch 8, unter Bedingungen, die die Bildung eines nachweisbaren Antikörper-Antigen-Komplexes erlauben; und
(b) das Nachweisen des Antikörper-Antigen-Komplexes, wobei die Anwesenheit des Antikörper-Antigen-Komplexes eine hohe Wahrscheinlichkeit anzeigt, dass das getestete Individuum Gelenkrheumatismus hat.

17. *In vitro*-Verfahren zum Identifizieren eines Individuums, das eine hohe Wahrscheinlichkeit aufweist, Gelenkrheumatismus zu haben, das umfasst:
(a) das Inkontaktbringen einer biologischen Probe mit dem Antikörper gemäß Anspruch 8, unter Bedingungen, die die Bildung eines nachweisbaren Antikörper-Antigen-Komplexes erlauben; und
(b) das Nachweisen des Antikörper-Antigen-Komplexes und das Vergleichen des Spiegels der Komplexe mit dem Spiegel von Antikörper-Antigen-Komplexen, die in den Proben, die von einem gesunden Individuum erhalten wurden, nachgewiesen wurden, wobei eine Abweichung von dem Spiegel eine hohe Wahrscheinlichkeit anzeigt, dass das getestete Individuum Gelenkrheumatismus hat.

18. Verfahren zur Identifizierung eines Peptids, das an das Polypeptid gemäß Anspruch 6 bindet, das umfasst:
(a) das Inkubieren von Zellen, die die Aminosäuresequenz exprimieren, mit einer Phagen-Display-Peptidbibliothek;
(b) das Waschen der Zellen, um ungebundene Phagen zu entfernen;
(c) das Eluieren von gebundenen Phagen aus den Zellen;
(d) das Amplifizieren des resultierenden gebundenen Phagen;
(e) das Ermitteln der Display-Peptidsequenz des gebundenen Phagen.

19. Verfahren zum Identifizieren von Kandidaten-Agonistverbindungen des Polypeptids gemäß Anspruch 6, das umfasst:
(a) das Bereitstellen eines Polypeptids, das eine Aminosäuresequenz wie dargestellt in SEQ ID NO: 2 umfasst;
(b) das Inkontaktbringen einer getesteten Kandidatenverbindung mit dem Polypeptid;
(c) das Messen des Effekts der Kandidatenverbindung auf die Aktivität des Polypeptids und das Auswählen einer Verbindung, die mindestens einen 70 %-induzierenden Effekt auf den Spiegel oder die Dauer der Aktivität zeigt.

20. Verfahren zum Identifizieren von Kandidaten-Antagonistverbindungen des Polypeptids gemäß Anspruch 6, das umfasst.
(a) das Bereitstellen eines Polypeptids, das eine Aminosäuresequenz wie dargestellt in SEQ ID NO: 2 umfasst;
(b) das Inkontaktbringen einer getesteten Kandidatenverbindung mit dem Polypeptid;
(c) das Messen des Effekts der Kandidatenverbindung auf die Aktivität des Polypeptids und das Auswählen einer Verbindung, die mindestens einen 70 %-inhibierenden Effekt auf den Spiegel oder die Dauer der Aktivität zeigt.

21. Verfahren zum Identifizieren einer Kandidatenverbindung, die eine Aktivität aufweist, die identisch ist zu der Aktivität des Polypeptids gemäß Anspruch 6, das umfasst:
(a) das Bereitstellen eines Polypeptids, das eine Aminosäuresequenz wie dargestellt in SEQ ID NO: 2 umfasst;
(b) das Messen der Aktivität des Polypeptids in einem Testassay;
(c) das Messen der Aktivität der Kandidatenverbindung in dem Testassay; und
(d) das Vergleichen der in (c) gemessenen Aktivität mit der in (b) gemessenen Aktivität, wobei eine in (c) gemessene Aktivität, die mindestens 70 % der in (b) gemessenen Aktivität ausmacht, anzeigt, dass die Kandidatenverbindung eine Verbindung mit einer Aktivität ist, die identisch zu der Aktivität des RA-CD44-Variante-Produkts ist.

22. Arzneimittel, das einen pharmazeutisch verträglichen Träger und ein Nucleinsäuremolekül gemäß Anspruch 1 oder 2, einen Expressionsvektor gemäß Anspruch 3 oder 4, eine Wirtszelle gemäß Anspruch 5, ein Polypeptid gemäß Anspruch 6 oder einen Antikörper gemäß einem der Ansprüche 8 bis 10 umfasst.

23. Arzneimittel gemäß Anspruch 22, zur Vorbeugung und Behandlung von Gelenkrheumatismus.

24. Verwendung eines Antikörpers gemäß einem der Ansprüche 8 bis 10 für die Herstellung eines Arzneimittels zur Vorbeugung oder Behandlung von Gelenkrheumatismus.

## Revendications

1. Molécule d'acide nucléique codant un variant de CD44 et qui comprend les trois nucléotides additionnels CAG ayant pour résultat une insertion dans un codon pour l'acide aminé alanine à l'extrémité 5' de l'exon v5 du gène de CD44, choisie dans le groupe constitué par :
(a) une molécule d'acide nucléique comprenant ou consistant en la séquence de la SEQ ID NO : 1 ;
(b) une molécule d'acide nucléique comprenant une séquence ayant une identité d'au moins 90 % avec la séquence de (a), ladite molécule d'acide nucléique comprenant les trois nucléotides additionnels CAG dans une région correspondant à la région de nucléotides 908-910 dans la SEQ ID NO : 1 ;
(c) une molécule d'acide nucléique qui est complémentaire de (a) ou (b) ;
(d) une molécule d'acide nucléique codant un polypeptide ayant la séquence d'acides aminés de la SEQ ID NO : 2 ; et
(e) une molécule d'acide nucléique codant un polypeptide ayant une séquence d'acides aminés identique à au moins 90 % à la SEQ ID NO : 2, ladite molécule d'acide nucléique comprenant les trois nucléotides additionnels CAG dans une région correspondant à la région de nucléotides 908-910 dans la SEQ ID NO : 1.

2. Fragment d'une molécule d'acide nucléique comprenant ou consistant en la séquence de la SEQ ID NO: 1 ayant au moins 20 nucléotides, ledit fragment comprenant les trois nucléotides additionnels CAG dans une région correspondant à la région de nucléotides 908-910 dans la SEQ ID NO : 1.

3. Vecteur d'expression comprenant la molécule d'acide nucléique selon la revendication 1 ou 2, conjointement avec des éléments de contrôle permettant l'expression desdites séquences d'acide nucléique dans une cellule hôte.

4. Vecteur d'expression selon la revendication 3, dans lequel lesdits éléments de contrôle permettent l'expression de ladite séquence d'acide nucléique dans une cellule hôte bactérienne ou dans une cellule hôte fongique.

5. Cellule hôte comprenant le vecteur d'expression selon la revendication 3 ou 4.

6. Polypeptide codé par la molécule d'acide nucléique selon la revendication 1 ou 2.

7. Fragment d'un polypeptide codé par une molécule d'acide nucléique comprenant la séquence de la SEQ ID NO : 1 ayant au moins 6 acides aminés, dans lequel ladite molécule d'acide nucléique comprend les trois nucléotides additionnels CAG dans une région correspondant à la région de nucléotides 908-910 dans la SEQ IL7 NO : 1, ledit fragment conservant le résidu d'acide aminé correspondant au résidu 303 de la SEQ ID NO : 2.

8. Anticorps qui se lie spécifiquement à :
(i) un épitope du polypeptide selon la revendication 6 ou 7, ledit épitope comprenant le résidu d'alanine correspondant à la position 303 de la SEQ ID NO : 2 ; et/ou
(ii) un néoépitope du polypeptide selon la revendication 6 ou 7, ledit néoépitope étant créé en conséquence du résidu d'alanine correspondant à la position 303 de la SEQ ID NO : 2.

9. Fragment de l'anticorps selon la revendication 8, ledit fragment conservant sensiblement les caractéristiques de liaison à un antigène de l'anticorps selon la revendication 6.

10. Anticorps selon la revendication 8 ou 9, ledit anticorps étant un anticorps monoclonal (mAb).

11. Lignée cellulaire immortalisée produisant des mAb selon la revendication 10.

12. Lignée cellulaire immortalisée selon la revendication 11, qui est une lignée cellulaire d'hybridome.

13. Procédé *in vitro* pour identifier un individu ayant une polyarthrite rhumatoïde, comprenant les étapes consistant à :
(a) disposer d'une sonde comprenant au moins l'une des molécules d'acide nucléique selon la revendication 1 ou 2 ;
(b) mettre en contact un échantillon biologique avec ladite sonde dans des conditions permettant l'hybridation de ladite sonde avec ledit produit de transcription pour former des complexes d'hybridation de sonde-produit de transcription détectables ;
(c) détecter les complexes d'hybridation de sonde-produit de transcription, la présence desdits complexes indiquant une forte probabilité que l'individu testé à partir duquel l'échantillon a été obtenu ait une polyarthrite rhumatoïde.

14. Procédé selon la revendication 13, dans lequel ladite sonde d'acide nucléique comprend une séquence détectant la présence d'un ARNm transcrit à partir de la séquence codante de la SEQ ID NO : 1.

15. Procédé *in vitro* pour identifier un individu ayant une polyarthrite rhumatoïde, comprenant les étapes consistant à :
(a) disposer d'une sonde comprenant au moins l'une des molécules d'acide nucléique selon la revendication 1 ou 2 ;
(b) mettre en contact un échantillon biologique avec ladite sonde dans des conditions permettant l'hybridation de ladite sonde avec ledit produit de transcription pour former des complexes d'hybridation de sonde-produit de transcription détectables ;
(c) détecter les complexes d'hybridation de sonde-produit de transcription et comparer le taux desdits complexes au taux des complexes d'hybridation détectés dans un échantillon biologique obtenu à partir d'un individu sain, un écart par rapport au taux des complexes d'hybridation dans ledit individu sain indiquant une forte probabilité que l'individu testé à partir duquel l'échantillon a été obtenu ait une polyarthrite rhumatoïde.

16. Procédé *in vitro* pour identifier un individu ayant forte probabilité d'avoir une polyarthrite rhumatoïde, consistant à :
(a) mettre en contact un échantillon biologique avec l'anticorps selon la revendication 8, dans des conditions permettant la formation d'un complexe d'anticorps-antigène détectable ; et
(b) détecter ledit complexe d'anticorps-antigène, la présence dudit complexe d'anticorps-antigène indiquant une forte probabilité que l'individu testé ait une polyarthrite rhumatoïde.

17. Procédé *in vitro* pour identifier un individu ayant forte probabilité d'avoir une polyarthrite rhumatoïde, consistant à :
(a) mettre en contact un échantillon biologique avec l'anticorps selon la revendication 8, dans des conditions permettant la formation d'un complexe d'anticorps-antigène détectable ; et
(b) détecter ledit complexe d'anticorps-antigène et comparer le taux desdits complexes avec le taux de complexes d'anticorps-antigène détectés dans des échantillons obtenus à partir d'un individu sain, un écart par rapport audit taux indiquant une forte probabilité que l'individu testé ait une polyarthrite rhumatoïde.

18. Procédé pour identifier un peptide qui se lie au polypeptide selon la revendication 6, consistant à :
(a) incuber des cellules exprimant ladite séquence d'acides aminés avec une bibliothèque de peptides de présentation sur phage ;
(b) laver les cellules pour éliminer les phages non liés ;
(c) éluer le phage lié à partir des cellules ;
(d) amplifier le phage lié résultant ;
(e) déterminer la séquence peptidique de présentation du phage lié.

19. Procédé pour identifier des composés agonistes candidats du polypeptide selon la revendication 6, consistant à :
(a) disposer d'un polypeptide comprenant une séquence d'acides aminés telle que décrite dans la SEQ ID NO : 2 ;
(b) mettre en contact un composé candidat de test avec ledit polypeptide ;
(c) mesurer l'effet dudit composé candidat sur l'activité dudit polypeptide et sélectionner un composé qui présente un effet inducteur d'au moins 70 % sur le taux ou la durée de ladite activité.

20. Procédé pour identifier des composés antagonistes candidats du polypeptide selon la revendication 6, consistant à :
(a) disposer d'un polypeptide comprenant une séquence d'acides aminés telle que décrite dans la SEQ ID NO : 2 ;
(b) mettre en contact un composé candidat de test avec ledit polypeptide ;
(c) mesurer l'effet dudit composé candidat sur l'activité dudit polypeptide et sélectionner un composé qui présente un effet inhibiteur d'au moins 70 % sur le taux ou la durée de ladite activité.

21. Procédé pour identifier un composé candidat ayant une activité qui est identique à l'activité du polypeptide selon la revendication 6, consistant à :
(a) disposer d'un polypeptide comprenant une séquence d'acides aminés telle que décrite dans la SEQ ID NO : 2 ;
(b) mesurer l'activité dudit polypeptide dans un dosage de test ;
(c) mesurer l'activité dudit composé candidat dans ledit dosage de test ; et
(d) comparer l'activité mesurée en (c) à l'activité mesurée en (b), une activité mesurée en (c) qui est d'au moins 70 % de l'activité mesurée en (b) indiquant que ledit composé candidat est un composé ayant une activité qui est identique à l'activité du produit variant de RA-CD44.

22. Composition pharmaceutique comprenant un véhicule acceptable en pharmacie, et une molécule d'acide nucléique selon la revendication 1 ou 2, un vecteur d'expression selon la revendication 3 ou 4, une cellule hôte selon la revendication 5, un polypeptide selon la revendication 6, ou un anticorps selon l'une quelconque des revendications 8 à 10.

23. Composition pharmaceutique selon la revendication 22, qui est destinée à la prévention et au traitement de la polyarthrite rhumatoïde.

24. Utilisation d'un anticorps selon l'une quelconque des revendications 8 à 10, pour la préparation d'une composition pharmaceutique destinée à la prévention ou au traitement de la polyarthrite rhumatoïde.


### SEQUENCE LISTING

<110> YISSUM RESEARCH DEVELOPMENT COMPANY
<120> NOVEL CD44 PEPTIDE
<130> NAOR David
<140>
   <141>
<160> 2
<170> PatentIn Ver. 2.1
<210> 1
   <211> 2100
   <212> DNA
   <212> DNA
   <213> HUMAN
<400> 1
   atggacaagt tttggtggca cgcagcctgg ggactctgcc tcgtgccgct gagcctggcg 60
   cagatcgatt tgaatataac ctgccgcttt gcaggtgtat tccacgtgga gaaaaatggt 120
   cgctacagca tctctcggac ggaggccgct gacctctgca aggctttcaa tagcaccttg 180
   cccacaatgg cccagatgga gaaagctctg agcatcggat ttgagacctg caggtatggg 240
   ttcatagaag ggcacgtggt gattccccgg atccacccca actccatctg tgcagcaaac 300
   aacacagggg tgtacatcct cacatccaac acctcccagt atgacacata ttgcttcaat 360
   gcttcagctc cacctgaaga agattgtaca tcagtcacag acctgcccaa tgcctttgat 420
   ggaccaatta ccataactat tgttaaccgt gatggcaccc gctatgtcca gaaaggagaa 480
   tacagaacga atcctgaaga catctacccc agcaacccta ctgatgatga cgtgagcagc 540
   ggctcctcca gtgaaaggag cagcacttca ggaggttaca tcttttacac cttttctact 600
   gtacacccca tcccagacga agacagtccc tggatcaccg acagcacaga cagaatccct 660
   gctaccagta cgtcttcaaa taccatctca gcaggctggg agccaaatga agaaaatgaa 720
   gatgaaagag acagacacct cagtttttct ggatcaggca ttgatgatga tgaagatttt 780
   atctccagca ccatttcaac cacaccacgg gcttttgacc acacaaaaca gaaccaggac 840
   tggacccagt ggaacccaag ccattcaaat ccggaagtgc tacttcagac aaccacaagg 900
   atgactgcag atgtagacag aaatggcacc actgcttatg aaggaaactg gaacccagaa 960
   gcacaccctc ccctcattca ccatgagcat catgaggaag aagagacccc acattctaca 1020
   agcacaatcc aggcaactcc tagtagtaca acggaagaaa cagctaccca gaaggaacag 1080
   tggtttggca acagatggca tgagggatat cgccaaacac ccagagaaga ctcccattcg 1140
   acaacaggga cagctgcagc ctcagctcat accagccatc caatgcaagg aaggacaaca 1200
   ccaagcccag aggacagttc ctggactgat ttcttcaacc caatctcaca ccccatggga 1260
   cgaggtcatc aagcaggaag aaggatggat atggactcca gtcatagtac aacgcttcag 1320
   cctactgcaa atccaaacac aggtttggtg gaagatttgg acaggacagg acctctttca 1380
   atgacaacgc agcagagtaa ttctcagagc ttctctacat cacatgaagg cttggaagaa 1440
   gataaagacc atccaacaac ttctactctg acatcaagca ataggaatga tgtcacaggt 1500
   ggaagaagag acccaaatca ttctgaaggc tcaactactt tactggaagg ttatacctct 1560
   cattacccac acacgaagga aagcaggacc ttcatcccag tgacctcagc taagactggg 1620
   tcctttggag ttactgcagt tactgttgga gattccaact ctaatgtcaa tcgttcctta 1680
   tcaggagacc aagacacatt ccaccccagt ggggggtccc ataccactca tggatctgaa 1740
   tcagatggac actcacatgg gagtcaagaa ggtggagcaa acacaacctc tggtcctata 1800
   aggacacccc aaattccaga atggctgatc atcttggcat ccctcttggc cttggctttg 1860
   attcttgcag tttgcattgc agtcaacagt cgaagaaggt gtgggcagaa gaaaaagcta 1920
   gtgatcaaca gtggcaatgg agctgtggag gacagaaagc caagtggact caacggagag 1980
   gccagcaagt ctcaggaaat ggtgcatttg gtgaacaagg agtcgtcaga aactccagac 2040
   cagtttatga cagctgatga gacaaggaac ctgcagaatg tggacatgaa gattggggtg 2100
<210> 2
   <211> 700
   <212> PRT
   <213> HUMAN
<400> 2
   Met Asp Lys Phe Trp Trp His Ala Ala Trp Gly Leu Cys Leu Val Pro
   1 5 10 15
   Leu Ser Leu Ala Gln Ile Asp Leu Asn Ile Thr Cys Arg Phe Ala Gly
   20 25 30
   Val Phe His Val Glu Lys Asn Gly Arg Tyr Ser Ile Ser Arg Thr Glu
   35 40 45
   Ala Ala Asp Leu Cys Lys Ala Phe Asn Ser Thr Leu Pro Thr Met Ala
   50 55 60
   Gln Met Glu Lys Ala Leu Ser Ile Gly Phe Glu Thr Cys Arg Tyr Gly
   65 70 75 80
   Phe Ile Glu Gly His Val Val Ile Pro Arg Ile His Pro Asn Ser Ile
   85 90 95
   Cys Ala Ala Asn Asn Thr Gly Val Tyr Ile Leu Thr Ser Asn Thr Ser
   100 105 110
   Gln Tyr Asp Thr Tyr Cys Phe Asn Ala Ser Ala Pro Pro Glu Glu Asp
   115 120 125
   Cys Thr Ser Val Thr Asp Leu Pro Asn Ala Phe Asp Gly Pro Ile Thr
   130 135 140
   Ile Thr Ile Val Asn Arg Asp Gly Thr Arg Tyr Val Gln Lys Gly Glu
   145 150 155 160
   Tyr Arg Thr Asn Pro Glu Asp Ile Tyr Pro Ser Asn Pro Thr Asp Asp
   165 170 175
   Asp Val Ser Ser Gly Ser Ser Ser Glu Arg Ser Ser Thr Ser Gly Gly
   180 185 190
   Tyr Ile Phe Tyr Thr Phe Ser Thr Val His Pro Ile Pro Asp Glu Asp
   195 200 205
   Ser Pro Trp Ile Thr Asp Ser Thr Asp Arg Ile Pro Ala Thr Thr Thr
   210 215 220
   Ser Ser Asn Thr Ile Ser Ala Gly Trp Glu Pro Asn Glu Glu Asn Glu
   225 230 235 240
   Asp Glu Arg Asp Arg His Leu Ser Phe Ser Gly Ser Gly Ile Asp Asp
   245 250 255
   Asp Glu Asp Phe Ile Ser Ser Thr Ile Ser Thr Thr Pro Arg Ala Phe
   260 265 270
   Asp His Thr Lys Gln Asn Gln Asp Trp Thr Gln Trp Asn Pro Ser His
   275 280 285
   Ser Asn Pro Glu Val Leu Leu Gln Thr Thr Thr Arg Met Thr Ala Asp
   290 295 300
   Val Asp Arg Asn Gly Thr Thr Ala Tyr Glu Gly Asn Trp Asn Pro Glu
   305 310 315 320
   Ala His Pro Pro Leu Ile His His Glu His His Glu Glu Glu Glu Thr
   325 330 335
   Pro His Ser Thr Ser Thr Ile Gln Ala Thr Pro Ser Ser Thr Thr Glu
   340 345 350
   Glu Thr Ala Thr Gln Lys Glu Gln Trp Phe Gly Asn Arg Trp His Glu
   355 360 365
   Gly Tyr Arg Gln Thr Pro Arg Glu Asp Ser His Ser Thr Thr Gly Thr
   370 375 380
   Ala Ala Ala Ser Ala His Thr Ser His Pro Met Gln Gly Arg Thr Thr
   385 390 395 400
   Pro Ser Pro Glu Asp Ser Ser Trp Thr Asp Phe Phe Asn Pro Ile Ser
   405 410 415
   His Pro Met Gly Arg Gly His Gln Ala Gly Arg Arg Met Asp Met Asp
   420 425 430
   Ser Ser His Ser Thr Thr Leu Gln Pro Thr Ala Asn Pro Asn Thr Gly
   435 440 445
   Leu Val Glu Asp Leu Asp Arg Thr Gly Pro Leu Ser Met Thr Thr Gln
   450 455 460
   Gln Ser Asn Ser Gln Ser Phe Ser Thr Ser His Glu Gly Leu Glu Glu
   465 470 475 480
   Asp Lys Asp His Pro Thr Thr Ser Thr Leu Thr Ser Ser Asn Arg Asn
   485 490 495
   Asp Val Thr Gly Gly Arg Arg Asp Pro Asn His Ser Glu Gly Ser Thr
   500 505 510
   Thr Leu Leu Glu Gly Tyr Thr Ser His Tyr Pro His Thr Lys Glu Ser
   515 520 525
   Arg Thr Phe Ile Pro Val Thr Ser Ala Lys Thr Gly Ser Phe Gly Val
   530 535 540
   Thr Ala Val Thr Val Gly Asp Ser Asn Ser Asn Val Asn Arg Ser Leu
   545 550 555 560
   Ser Gly Asp Gln Asp Thr Phe His Pro Ser Gly Gly Ser His Thr Thr
   565 570 575
   His Gly Ser Glu Ser Asp Gly His Ser His Gly Ser Gln Glu Gly Gly
   580 585 590
   Ala Asn Thr Thr Ser Gly Pro Ile Arg Thr Pro Gln Ile Pro Glu Trp
   595 600 605
   Leu Ile Ile Leu Ala Ser Leu Leu Ala Leu Ala Leu Ile Leu Ala Val
   610 615 620
   Cys Ile Ala Val Asn Ser Arg Arg Arg Cys Gly Gln Lys Lys Lys Leu
   625 630 635 640
   Val Ile Asn Ser Gly Asn Gly Ala Val Glu Asp Arg Lys Pro Ser Gly
   645 650 655
   Leu Asn Gly Glu Ala Ser Lys Ser Gln Glu Met Val His Leu Val Asn
   660 665 670
   Lys Glu Ser Ser Glu Thr Pro Asp Gln Phe Met Thr Ala Asp Glu Thr
   675 680 685
   Arg Asn Leu Gln Asn Val Asp Met Lys Ile Gly Val
   690 695 700